# EUROPEAN PATENT APPLICATION

(11) **EP 4 850 777 A1**
(43) Date of publication of application: **09.12.2026**
(21) Application number: 24921508.8
(22) Date of filing: 11.10.2024
(51) Int. Cl.: B01D 3/00, B01J 8/02, B01J 19/32

(54) **CATALYTIC PACKING MODULE AND USE THEREOF**

(30) Priority: 31.01.2024 CN 202410137325
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: QIAO, Kai, Dalian, Liaoning 116045 (CN); SUN, Qimei, Dalian, Liaoning 116045 (CN); ZHOU, Feng, Dalian, Liaoning 116045 (CN); BAI, Yuli, Dalian, Liaoning 116045 (CN); LI, Lanpeng, Dalian, Liaoning 116045 (CN)
(74) Representative: karo IP
(86) International application number: PCT/CN2024/124063
(87) International publication number: WO 2025/161484

(57) **Abstract**

It is disclosed a catalytic packing module suitable for a catalytic distillation apparatus, which comprises, sequentially from the inside out, a solid catalyst layer at the center, a first mesh layer wrapped around the outside of the catalyst layer, an inert packing layer wrapped around the outside of the first mesh layer, and a second mesh layer wrapped around the outside of the inert packing layer. The catalytic packing module employs a special layered structural design, ensuring adequate contact between the reaction feedstock and the catalyst, while also offering the advantages of ease of loading and unloading, operational stability, low catalyst attrition, and the capability to adjust the catalyst loading as needed.

## Description

### Technical Field

The present application relates to the technical field of reactive distillation, specifically to a catalytic packing module and the use thereof.

### Background Technology

Polylactic acid, also known as polylactide (PLA), is produced by fermenting a renewable biomass feedstock such as corn, cassava, straw and the like to generate lactic acid monomers, and then polymerizing the monomers through a series of reactions. It exhibits good mechanical properties, processability, and biodegradability. After use, PLA products can be composted and degraded into CO₂ and water, completing a natural cycle. Currently, the main production process for polylactic acid involves using lactic acid as the raw material, first preparing lactide, and then polymerizing lactide via ring-opening polymerization to obtain polylactic acid, as shown in the following equation.

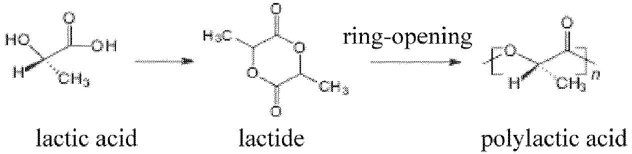

As the key core of the entire polylactic acid industry chain, there are currently two main methods for synthesizing lactide. One is the "two-step method", which is the current industrialized production process; specifically, lactic acid is polymerized to generate lactic acid multimers, and then the multimers are depolymerized to generate lactide. The other is the "one-step method", in which lactic acid is synthesized into lactide in one step under the action of a catalyst.

The "one-step method" for producing lactide features low energy consumption, does not require negative-pressure operation, and has a relatively simple process route. However, it has drawbacks such as relatively low yield, uncontrollable process, susceptibility of the product to hydrolysis in the aqueous reaction system, and significant challenges in continuous industrial production. There are two approaches for the "one-step method" for production of lactide. One is the liquid-phase one-step reaction process (as described, for example, in the 2015 article "Shape-selective zeolite catalysis for bioplastics production" by M. Dusselier's team at KU Leuven, published in Science, Vol. 349, and the 2020 article "Breaking the Si/Al limit of nanosized β Zeolites: promoting catalytic production of lactide" by Jilin University and the Changchun Institute of Applied Chemistry, Chinese Academy of Sciences, published in Chemistry of Materials, Vol. 32, Issue 2). This approach operates at a relatively lower reaction temperature and offers a higher product yield and selectivity, but it is currently mostly carried out in batch reactors, making scale-up difficult. The other is the gas-phase one-step reaction process (as described, for example, in DuPont's patent US5043458), which operates at a relatively higher reaction temperature, typically in fixed-bed or fluidized-bed reactors. This approach suffers from a low product yield and relatively severe racemization, but it allows for continuous operation.

Catalytic distillation integrates the reaction and separation processes into a single apparatus, offering advantages such as low energy consumption and minimal investment. It can overcome the limitations of chemical equilibrium, improve reaction selectivity, and theoretically play a positive role in promoting the direct cyclization of lactic acid to lactide and the timely separation of reaction water. The key to catalytic distillation technology lies in the configuration of the catalytic distillation components within the catalytic distillation apparatus. Among these, the packing configuration and the packing mode of the catalyst and packing material-i.e., the catalytic packing module or assembly-are one of the crucial influencing factors. Existing technologies have already undertaken some work on catalytic distillation modules or assemblies, and some devices for enhancing catalytic reactive distillation have emerged by combining gas-liquid-solid three-phase reaction systems.

US Patent US4443559 discloses a catalytic distillation structure for a catalytic distillation reaction section. It packs the catalyst into small bags with mesh to form catalyst bundles, and then rolls it up with an elastic component to form a cylindrical catalytic distillation component with dual functions of catalytic reaction and distillation separation. This component is then filled into a catalytic distillation apparatus. The disadvantage of this filling method is that the gas-liquid distribution in the column is uneven, which affects the coupling of the two effects of distillation and separation.

Both US Patent US52621012 and Chinese Patent CN1042664A disclose a method of loading catalyst in bulk form into a catalytic distillation reaction section. The obvious drawback of this loading method is that the gas-liquid distribution in the column is uneven, which can easily cause catalyst attrition and affect the conversion rate.

For the one-step cyclization of lactic acid in liquid phase to prepare lactide, the continuous reaction process, high selectivity, and high yield are the current technological challenges. The inventors of the present application discovered that the reaction process can be continuously carried out by combining the characteristics of a catalytic distillation apparatus and adjusting the scheme. However, improving reaction selectivity and reducing the probability of lactide hydrolysis are the key point. Therefore, it is necessary to develop a novel catalytic packing module suitable for catalytic distillation apparatus.

### Content of the Invention

In view of the above-mentioned technical problems and the shortcomings of the prior art, the object of the present application is to provide a novel catalytic packing module and the use thereof. The catalytic packing module employs a special layered structural design, ensuring adequate contact between the reaction feedstock and the catalyst, while also offering the advantages of ease of loading and unloading, operational stability, low catalyst attrition, and the capability to adjust the catalyst loading as needed.

To achieve the above object, on one aspect, the present application provides a catalytic packing module suitable for a catalytic distillation apparatus comprising, sequentially from the inside out, a solid catalyst layer at the center, a first mesh layer wrapped around the outside of the catalyst layer, an inert packing layer wrapped around the outside of the first mesh layer, and a second mesh layer wrapped around the outside of the inert packing layer.

Preferably, the first mesh layer and the second mesh layer are made of corrosion-resistant materials having different hydrophilic and/or lipophilic properties.

More preferably, the first mesh layer is made of a hydrophobic and corrosion-resistant material, and the second mesh layer is made of a highly permeable and hydrophilic corrosion-resistant material.

On another aspect, the present application provides a structured catalytic packing comprising catalytic packing modules and packing modules arranged alternately in a horizontal direction, wherein the catalytic packing modules are the catalytic packing modules according to the present application.

On another aspect, the present application provides a catalytic distillation apparatus comprising an upper rectification section and a lower reaction section, with a feed inlet provided between the upper rectification section and the lower reaction section, wherein the lower reaction section is packed with at least one layer of the structured catalytic packing according to the present application.

Preferably, the lower reaction section of the catalytic distillation apparatus is packed with multiple layers of the structured catalytic packing according to the present application, and in the vertical direction, the catalytic packing modules and the packing modules of two adjacent layers of the structured catalytic packing among the multiple layers are arranged in a staggered manner vertically.

On yet another aspect, it is provided use of the catalytic packing module, the structured catalytic packing, or the catalytic distillation apparatus according to the present application in a solid-phase catalytic reaction of a liquid feedstock, particularly in a reaction process for preparing lactide from lactic acid via a one-step liquid-phase method.

In yet another aspect, the present application provides a catalytic distillation reaction method comprising the steps of: in the reaction section of the catalytic distillation apparatus according to the present application, bringing a liquid feedstock into contact with a solid catalyst arranged within the catalytic packing module for reaction, and separating a resulting reaction product in the rectification section.

On yet another aspect, the present application provides a one-step continuous process for preparing lactide, comprising the step of: in the catalytic distillation apparatus according to the present application, bringing lactic acid into contact with a catalyst for reaction to obtain lactide, wherein a feedstock containing lactic acid enters through the feed inlet of the catalytic distillation apparatus, comes into contact with the catalyst within the catalytic packing modules in the reaction section for reaction, an reaction inert gas enters through a gas inlet at the bottom of the reaction section and passes upward through the reaction section, stripping a portion of water and a portion of unreacted lactic acid from the reaction materials in the reaction section into the rectification section, and a crude lactide product is withdrawn from a product outlet at the bottom of the reaction section.

Compared with the prior art, the present application has at least one of the following beneficial effects:
(1) The catalytic packing module of the present application employs a special layered structural design, in which the catalyst and the inert packing materials are separately wrapped in layers. This allows the use of fine catalyst particles, provides sufficient gas-liquid contact area for the reaction, and ensures adequate contact between the reaction materials and the catalyst. In addition, it offers advantages such as ease of loading and unloading, stable operation, low catalyst attrition, and the capability to adjust the catalyst amount as needed.
(2) The first and second mesh layers of the catalytic packing module of the present application can be made of materials with different properties, thereby enabling timely separation of target products and by-product components with different polarities or hydrophilicities, and reducing the probability of reverse reactions between them.
(3) In the structured catalytic packing of the present application, the catalytic packing modules and packing modules are arranged alternately in the horizontal direction. This arrangement not only achieves a uniform distribution of the catalyst across the cross-section of the reaction section of the catalytic distillation apparatus, but also increases liquid holdup, achieving sufficient contact between the reaction materials and the catalyst. Meanwhile, when multiple layers of structured catalytic packing are used in combination and in the vertical direction, the catalytic packing modules and packing modules of two adjacent layers of structured catalytic packing are arranged in a staggered manner vertically, it can not only further achieve uniform distribution of the catalyst throughout the entire reaction section of the catalytic distillation apparatus in both the transverse and longitudinal directions, but also reduce the bed pressure drop, so that the generated light by-products can be removed from the reaction system in a timely manner.
(4) In the present application, the catalytic packing module with the special layered structure is applied to a catalytic distillation apparatus and used in the continuous one-step liquid-phase production of lactide from lactic acid. This can improve heat and mass transfer, reduce column pressure drop, and enable the steady, orderly and timely removal of free water from the reaction process and intermolecular bound water generated during the reaction. This ensures the stable cyclization reaction of lactic acid dimers and trimers on the catalyst, avoids rapid re-hydrolysis of lactide in the presence of water at high temperatures, improves reaction efficiency, and allows for continuous and stable operation of the reaction process, thereby enabling continuous production of lactide.
(5) When the catalytic distillation apparatus of the present application is applied to the liquid-phase cyclization of lactic acid to produce lactide, it can also effectively avoid the process steps of separating the catalyst from the reaction system and activating in a traditional tank reactor. This significantly extends the service life of the catalyst and improves catalyst utilization. The single-pass yield of lactide can reach 80% or higher, the chemical purity of crude lactide can reach 78% or higher (up to 88%), and the content of m-lactide can be controlled within 2.0%.

Other features and advantages of the present application will be described in detail in the following part of Specific Embodiments.

### Description of the Accompanying Drawings

The accompanying drawings are provided to further illustrate the present application and form part of the specification. They are used together with the following specific embodiments to explain the present application, but do not constitute a limitation thereof. In the accompanying drawings:
Figure 1 shows a cross-sectional view of a preferred embodiment of the catalytic packing module of the present application;
Figure 2 shows a cross-sectional view of a preferred embodiment of the structured catalytic packing of the present application, and a cross-sectional view of an adjacent structured catalytic packing when the multilayer structured catalytic packings are arranged in a longitudinally staggered manner.
Figure 3 shows a cross-sectional view of another preferred embodiment of the structured catalytic packing of the present application, and a cross-sectional view of an adjacent structured catalytic packing when the multilayer structured catalytic packings are arranged in a longitudinally staggered manner.
Figure 4 shows a schematic diagram of a preferred embodiment of the catalytic distillation apparatus of the present application;
Figure 5 shows a schematic diagram of a preferred embodiment of the first and second mesh layers of the catalytic packing module of the present application; and
Figure 6 shows a schematic diagram of another preferred embodiment of the first and second mesh layers of the catalytic packing module of the present application.

### Explanation of reference numerals in the accompanying drawings

101 Second mesh layer, 102 Inert packing layer, 103 First mesh layer, 104 Catalyst particle layer, 201 Catalytic packing module, 202 Packing module, 203 Inner wall of catalytic distillation apparatus, 401 Catalytic distillation apparatus, 411 Rectification section, 412 Reaction section, 413 Top condenser.

### Specific Embodiments

The specific embodiments of the present application will be described in detail below with reference to the accompanying drawings. It should be understood that the specific embodiments described herein are for illustration and explanation only and are not intended to limit the present application.

Any specific numerical values disclosed herein (including the endpoints of numerical ranges) are not limited to their exact values, but should be understood to also include values close to the exact values, such as all possible values within ± 5% of the exact values. Furthermore, with respect to the disclosed numerical ranges, one or more new numerical ranges can be obtained by arbitrarily combining the endpoint values of the range, the endpoint values with specific point values within the range, and the specific point values themselves; these new numerical ranges should also be considered as specifically disclosed herein.

Unless otherwise stated, the terms used herein have the same meaning as commonly understood by those skilled in the art, and if a term is defined herein and its definition differs from the common understanding in the art, the definition herein shall prevail.

In the present application, except where expressly stated, any matters or issues not mentioned are directly applicable to aspects known in the art without any modification. Furthermore, any embodiments described herein can be freely combined with one or more other embodiments described herein, and the resulting technical solutions or concepts are considered part of the original disclosure or original record of the present application, and should not be regarded as new content not disclosed or anticipated herein, unless those skilled in the art consider the combination apparently unreasonable.

All patent and non-patent literature mentioned herein, including but not limited to textbooks and journal articles, are incorporated as a whole by way of citation.

As described above, in a first aspect, the present application provides a catalytic packing module suitable for a catalytic distillation apparatus comprising, sequentially from the inside out, a solid catalyst layer at the center, a first mesh layer wrapped around the outside of the catalyst layer, an inert packing layer wrapped around the outside of the first mesh layer, and a second mesh layer wrapped around the outside of the inert packing layer.

The catalytic packing module of the present application adopts a special layered structure design, in which the solid catalyst and the inert packing materials are separately wrapped in layers, which allows the use of fine particulate catalyst, provides sufficient gas-liquid contact area for the reaction, and ensures adequate contact between the reaction materials and the catalyst.

In a preferred embodiment, the first and second mesh layers are made of corrosion-resistant materials having different hydrophilic and/or lipophilic properties. More preferably, the first mesh layer (also called the inner mesh layer), which wraps around and contacts the catalyst layer, is made of a hydrophobic and corrosion-resistant material, such as a polyester, a fluoropolymer, or a hydrophobic metal-based composite material. These materials include, but are not limited to, polycarbonate, polyvinylidene fluoride, fluorinated amphiphilic polymers, and hydrophobic titanium-based composite materials, and the like. Furthermore, the second mesh layer (also called the outer layer), which wraps around and contacts the inert packing layer, is made of a highly permeable and hydrophilic corrosion-resistant material, such as a highly permeable hydrophilic fiber coalescence material. These materials include, but are not limited to, glass fiber fabrics, surface-coated, modified, or grafted PVDF materials, and TiO₂ materials treated with SiO₂ compositing or surface chemical modifications such as ion doping.

In the preferred embodiment described above, the first and second mesh layers of the catalytic packing module are made of materials with different properties, which can promptly separate the target reaction product and by-product components with different polarities or hydrophilicities, reducing the probability of reverse reactions between the two. For example, when used in the one-step liquid-phase method for preparing lactide from lactic acid, the catalytic packing module can ensure that lactic acid dimers and trimers quickly enter the catalyst pores to react, and can also ensure that the generated small water molecules leave the reaction system in a timely manner and aggregate into small droplets on the outer surface of the second mesh layer for rapid removal. This reduces the probability of reverse reactions caused by contact between water and lactide, and improves the efficiency of the one-step liquid-phase reaction.

According to the present application, the first and second mesh layers can be various conventionally used mesh structures, and can be selected according to actual application needs. The present application does not have strict limitations on this, as long as it allows liquid to pass freely while preventing the passage of solid catalysts and inert packing materials. In a preferred embodiment, the first and second mesh layers are each independently selected from mesh materials such as corrugated mesh and wire mesh. Furthermore, the first and second mesh layers themselves can be one or more layers, specifically selected according to actual application needs, and the present application does not have strict limitations in this regard.

According to the present application, the mesh shapes of the first and second mesh layers can be various conventional shapes, including but not limited to square, circular, rhomboid, triangular, hexagonal, oblong, and plum blossom shapes, etc., and can be selected according to actual application needs. The present application does not have strict limitations in this regard. In a preferred embodiment, the mesh shapes of the first and second mesh layers are each independently selected from circular and oblong shapes.

According to the present application, the porosity and mesh size of the first and second mesh layers can be selected according to the actual application needs, and the present application does not impose strict limitations in this regard. In a preferred embodiment, the porosity of the first and second mesh layers is independently 50.0-70.0%, and the mesh size is independently 7-30 mesh, preferably 10-25 mesh.

In the catalytic packing module of the present application, the inert packing material can separate the first and second mesh layers, especially the first and second mesh layers composed of different materials, and help to enrich different reaction products (e.g., water and lactide) at the first and second mesh layers respectively, reducing the probability of contact between the two reaction products, lowering the probability of reverse reaction, and playing a role in product separation. According to the present application, the inert packing material can be the inert packing material commonly used in various catalytic distillation apparatuses, and can be selected according to actual application needs; the present application does not have strict limitations in this regard. In a preferred embodiment, the inert packing material is selected from glass beads, ceramic rings, ceramic spheres, ceramic skeletons, quartz sand, silica sand, bentonite, or combinations thereof. More preferably, the particle size of the packing material is 1.0-2.0 mm.

According to the present application, the solid catalyst contained in the catalytic packing module can be selected according to the specific catalytic reaction to be applied, and the present application does not impose strict limitations in this regard. For example, when used in the one-step liquid-phase method for preparing lactide from lactic acid, the catalyst contained in the catalytic packing module can be any conventionally used solid catalyst capable of catalyzing the liquid-phase reaction of lactic acid to produce lactide, preferably a catalyst based on a molecular sieve loaded with Sn and/or Ti, preferably a catalyst based on a β molecular sieve loaded with Sn and/or Ti, particularly preferably a catalyst based on a β molecular sieve loaded with Sn and Ti. The catalyst can be prepared by various known catalyst preparation methods, such as conventional impregnation and loading methods, and the present application does not impose strict limitations in this regard.

According to the present application, the catalyst can be of various shapes and structures commonly used in catalytic distillation apparatus, and can be selected according to actual application needs; the present application does not impose strict limitations in this regard. In a preferred embodiment, the catalyst is in the form of spheres or porous spheres having a diameter of 1-5 mm, cylinders or porous cylinders having a diameter of 0.5-3 mm and a length of 1-5 mm, or cloverleaf shapes or polyhedral shapes having an equivalent diameter of 1-5 mm. The catalyst can be formed into the desired shape and structure using various known catalyst forming methods, such as powder extrusion molding process.

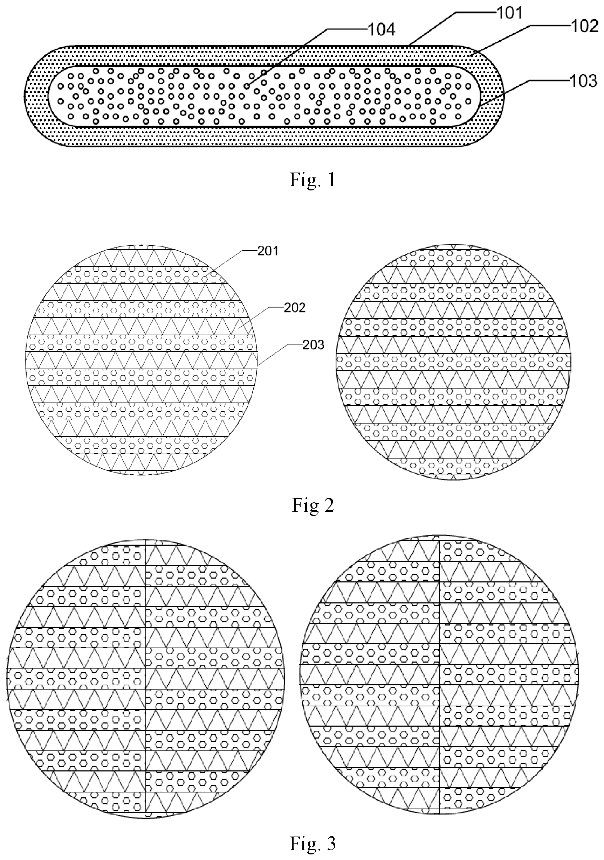

Figure 1 shows a schematic diagram of a preferred embodiment of the catalyst packing module of the present application, wherein the module comprises, sequentially from the inside out, a catalyst particle layer 104 located at the center, a first mesh layer 103 wrapped around the outside of the catalyst layer, an inert packing layer 102 wrapped around the outside of the first mesh layer, and a second mesh layer 101 wrapped around the outside of the inert packing layer.

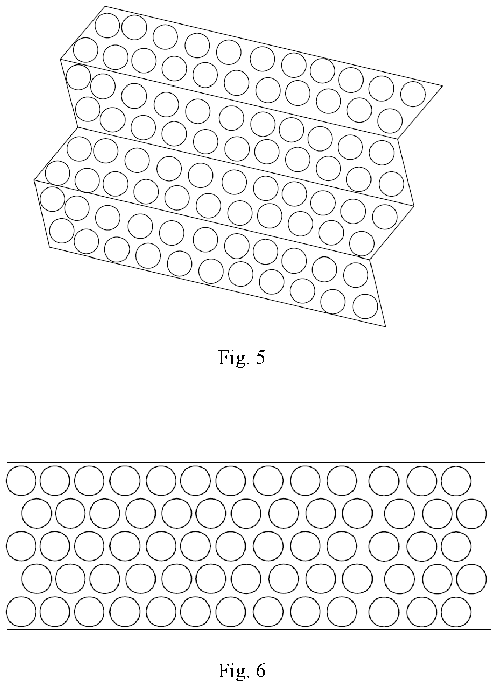

Figure 5 shows a schematic diagram of a preferred embodiment of the first and second mesh layers of the catalytic packing module of the present application. As shown, in this preferred embodiment, the first mesh layer and/or the second mesh layer is in the form of a circular hole corrugated mesh.

Figure 6 shows a schematic diagram of another preferred embodiment of the first and second mesh layers of the catalytic packing module of the present application. As shown, in this preferred embodiment, the first mesh layer and/or the second mesh layer is in the form of a circular hole wire mesh.

In a second aspect, the present application provides a structured catalytic packing, comprising catalytic packing modules and packing modules arranged alternately in a horizontal direction, wherein the catalytic packing modules are the catalytic packing modules of the present application.

According to the present application, the packing module can be of the structure and form of packing modules commonly used in various catalytic distillation apparatuses, and can be selected according to the actual application needs; the present application does not impose strict limitations in this regard. In a preferred embodiment, the packing module is composed of stainless steel wire mesh corrugated packing, which can provide support for adjacent catalytic packing modules.

According to the present application, in the structured catalytic packing, the catalytic packing modules and packing modules are arranged alternately in the horizontal direction, for example, arranged alternately in the form of "catalytic packing module || packing module || catalytic packing module || packing module". The specific arrangement can be selected according to the actual application needs. The present application does not have strict restrictions in this regard, as long as it can be achieved that any two catalytic packing modules are not adjacent to each other and that any two packing modules are not adjacent to each other in the horizontal direction.

Figure 2 shows a schematic diagram of a preferred embodiment of the structured catalytic packing of the present application, wherein the catalytic packing module 201 and the packing module 202 are arranged alternately in a horizontal direction.

Figure 3 shows a schematic diagram of another preferred embodiment of the structured catalytic packing of the present application, wherein the catalytic packing modules and the packing modules are arranged alternately in two horizontal directions.

In the structured catalytic packing of the present application, the alternating arrangement of the catalytic packing modules and packing modules in the horizontal direction can achieve uniform distribution of the catalyst in the cross-section of the reaction section of the catalytic distillation apparatus, increase the liquid holdup, and achieve adequate contact between the reaction materials and the catalyst. At the same time, when multiple layers of structured catalytic packing are used in combination, and in the vertical direction, the catalytic packing modules and packing modules of two adjacent layers of structured catalytic packing are arranged in a staggered manner vertically, it can further achieve uniform distribution of the catalyst in the entire reaction section of the catalytic distillation apparatus in both the horizontal and vertical directions, and can also reduce the bed pressure drop, so that the generated light by-products can be removed from the reaction system in a timely manner.

In a third aspect, the present application provides a catalytic distillation apparatus, comprising an upper rectification section and a lower reaction section, with a feed inlet provided between the upper rectification section and the lower reaction section, wherein the lower reaction section is packed with at least one layer of the structured catalytic packing of the present application.

In a preferred embodiment, the lower reaction section of the catalytic distillation apparatus is packed with multiple layers, preferably 4-16 layers, of the structured catalytic packing of the present application, and in the vertical direction, the catalytic packing modules and the packing modules of two adjacent layers of the structured catalytic packing among the multiple layers are arranged in a staggered manner vertically.

In this preferred embodiment, in the catalytic distillation apparatus, in the vertical direction, the catalytic packing modules and the packing modules of two adjacent layers of structured catalytic packing are arranged in a staggered manner vertically, resulting in an alternating arrangement of the catalytic packing modules and the packing modules of the multilayer structured catalytic packing in the vertical direction. That is, above the catalytic packing module of a lower layer of structured catalytic packing is the packing module of the adjacent upper layer of structured catalytic packing, while above the packing module of the lower layer of structured catalytic packing is the catalytic packing module of the adjacent upper layer of structured catalytic packing, and so on. This ensures uniform distribution of the catalyst throughout the entire reaction section of the catalytic distillation apparatus in both the transverse and longitudinal directions, increases liquid holdup, and achieves sufficient contact between the reaction materials and the catalyst. It also reduces the pressure drop across the entire reaction section, so that the generated light by-products can be removed from the reaction system in a timely manner. For example, when used in the one-step liquid-phase continuous preparation of lactide from lactic acid, the catalytic distillation apparatus increases liquid holdup, ensuring sufficient contact between the lactic acid multimers and the catalyst, while reducing the pressure drop across the entire reaction section, promoting the timely escape of water from the viscous system, and improving reaction efficiency.

Figures 2 and 3 illustrate a preferred embodiment of the multilayer structured catalytic packing arrangement in the catalytic distillation apparatus of the present application, wherein the structured catalytic packing on the left and the structured catalytic packing on the right are arranged adjacent to each other vertically, such that the catalytic packing modules and packing modules of the lower layer of structured catalytic packing are staggered vertically with the catalytic packing modules and packing modules of the upper layer of structured catalytic packing.

According to the present application, the upper rectification section of the catalytic distillation apparatus can be equipped with various internal components and/or packings commonly used in the rectification sections of catalytic distillation apparatuses, and the selection can be made according to the actual application needs. The present application does not impose strict limitations in this regard. For example, in some preferred embodiments, the upper rectification section of the catalytic distillation apparatus of the present application is filled with structured packing selected from titanium wire mesh corrugated packing, perforated plate corrugated packing, or combinations thereof, to separate light components from entrained relatively heavy components.

In a preferred embodiment, the catalytic distillation apparatus further includes a condenser located at the top, which can cool and separate the vapor phase at the top of the apparatus. For example, in the one-step liquid-phase preparation of lactide from lactic acid, it can achieve rapid separation of water and non-condensable vapor (reaction inert gas). The resulting non-condensable vapor obtained through separation can be directly recycled after dehydration and heating.

In a preferred embodiment, the catalytic distillation apparatus further includes a gas inlet and a product outlet located at the bottom of the reaction section.

Optionally, as needed, a feed distributor may be provided at the feed inlet position of the catalytic distillation apparatus, and/or a gas distributor may be provided at the gas inlet position, for uniformly distributing the feed and/or gas across the cross-section of the reaction section.

Optionally, as needed, a reboiler may also be provided at the bottom of the catalytic distillation apparatus.

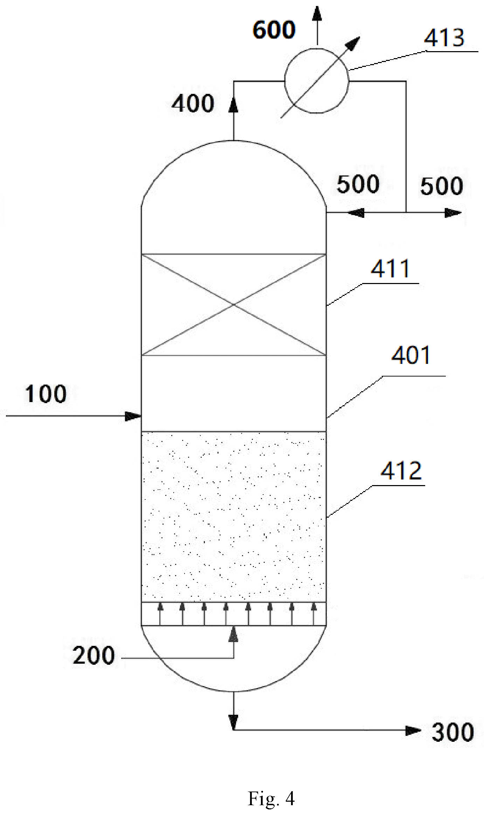

Figure 4 shows a schematic diagram of a preferred embodiment of the catalytic distillation apparatus of the present application, wherein the catalytic distillation apparatus 401 is in the form of a catalytic distillation column, including an upper rectification section 411 and a lower reaction section 412. The reaction section 412 is filled with multiple layers of the structured catalytic packing of the present application. A feed inlet is provided between the upper rectification section 411 and the lower reaction section 412. The catalytic distillation apparatus also includes a condenser 413 disposed at the top, and a gas inlet and a product outlet disposed at the bottom of the reaction section. Optionally, a gas distributor may also be provided at the gas inlet position.

In a fourth aspect, it is provided use of the catalytic packing module, the structured catalytic packing, or the catalytic distillation apparatus according to the present application in a solid-phase catalytic reaction of a liquid feedstock, particularly in a reaction process for preparing lactide from lactic acid via a one-step liquid-phase method.

The catalytic packing module, the structured catalytic packing, or the catalytic distillation apparatus according to the present application are suitable for various solid-phase catalytic reactions of liquid feedstock that are suitable for being carried out by catalytic distillation, such as the synthesis of lactic acid esters, the synthesis of isosorbide, and the synthesis of furanyl dicarboxylic acid, and the like.

In a preferred embodiment, the first and second mesh layers of the catalytic packing module are made of corrosion-resistant materials with different hydrophilic and/or lipophilic properties.

In a further preferred embodiment, the first mesh layer of the catalytic packing module is made of a hydrophobic and corrosion-resistant material, such as a polyester, a fluoropolymer or a metal composite material, particularly is made of a polyvinylidene fluoride; and the second mesh layer of the catalytic packing module is made of a highly permeable and hydrophilic corrosion-resistant material, such as a highly permeable hydrophilic fiber coalescence material, particularly made of glass fiber fabric.

In a fifth aspect, it is provided a catalytic distillation reaction method comprising the steps of: in the reaction section of the catalytic distillation apparatus according to the present application, bringing a liquid feedstock into contact with a solid catalyst arranged within the catalytic packing module for reaction, and separating a resulting reaction product in the rectification section.

In a preferred embodiment, the first and second mesh layers of the catalytic packing module are made of corrosion-resistant materials having different hydrophilic and/or lipophilic properties.

In a sixth aspect, it is provided a one-step continuous process for preparing lactide, comprising the step of: in the catalytic distillation apparatus of the present application, bringing lactic acid into contact with a catalyst for reaction to obtain lactide, wherein a feedstock containing lactic acid enters through the feed inlet of the catalytic distillation apparatus, comes into contact with the catalyst within the catalytic packing modules in the reaction section for reaction, a reaction inert gas enters through the gas inlet at the bottom of the reaction section and passes upward through the reaction section, stripping a portion of water and a portion of unreacted lactic acid from the reaction materials in the reaction section into the rectification section, and a crude lactide product is withdrawn from the product outlet at the bottom of the reaction section.

In a preferred embodiment, the first mesh layer of the catalytic packing module is made of a hydrophobic and corrosion-resistant material, such as a polyester, a fluoropolymer or a metal composite material, particularly is made of a polyvinylidene fluoride; and the second mesh layer of the catalytic packing module is made of a highly permeable and hydrophilic corrosion-resistant material, such as a highly permeable hydrophilic fiber coalescence material, particularly made of glass fiber fabric.

In a preferred embodiment, the rectification section of the catalytic distillation apparatus in the method has a theoretical plate number of 4 to 8, which can sufficiently separate lactic acid entrained in the gas phase and improve the reaction conversion rate.

In a preferred embodiment, the reaction section of the catalytic distillation apparatus in the method has a theoretical plate number of 2 to 8, which can sufficiently promote the intermolecular esterification reaction of lactic acid to generate lactic acid multimers and ensure that lactic acid dimers and trimers are sufficiently cyclized to generate lactide, thereby improving product yield.

In a preferred embodiment, the reflux ratio of the rectification section of the catalytic distillation apparatus in the method is from 2:1 to 1:5, for example, 1:2 to 1: 5, to ensure stable operation of the purification process in the rectification section of the catalytic reaction apparatus. More preferably, the operating conditions within the rectification section of the catalytic distillation apparatus further include: an overhead temperature of 95 to 140°C, and an overhead pressure, in absolute pressure, of 0.02 to 0.1 MPa.

In a preferred embodiment, the catalyst within the catalytic packing module is a catalyst based on a molecular sieve loaded with Sn and/or Ti, more preferably a catalyst based on a β molecular sieve loaded with Sn and/or Ti, particularly preferably a catalyst based on a β molecular sieve loaded with Sn and Ti.

In a preferred embodiment, the catalyst loading amount in the catalytic packing modules in the reaction section of the catalytic distillation apparatus is 0.1 to 0.6 cm³, preferably 0.2 to 0.6 cm³, more preferably 0.3 to 0.6 cm³ of catalyst volume per cm³ of reaction section volume.

In a preferred embodiment, the inert packing material loading amount in the catalytic packing modules in the reaction section of the catalytic distillation apparatus is 0.05 to 0.3 cm³, preferably 0.1 to 0.3 cm³, more preferably 0.1-0.25 cm³ of inert packing material per cm³ of reaction section volume.

In a preferred embodiment, the pressure in the reaction section of the catalytic distillation apparatus in the method is, in absolute pressure, from 0.05 to 0.15 MPa, preferably 0.08 to 0.12 MPa.

In a preferred embodiment, the reaction temperature in the reaction section of the catalytic distillation apparatus in the method is 100-160°C, preferably 120-140 °C, and the residence time of the feedstock containing lactic acid in the reaction section is 20-60 min.

In a preferred embodiment, the feedstock containing lactic acid in the method is a 30-60 wt% aqueous solution of lactic acid, with a feed temperature of 80-100°C.

In the method of the present application, the reaction inert gas refers to a gas that does not affect the reaction process, such as nitrogen. The inert gas is introduced from the bottom of the reaction section to strip the free or bound water in the reaction section, thus avoiding the use of organic solvents. The pressure of the inert gas is slightly higher than the reaction pressure to ensure its smooth upward movement. In a preferred embodiment, the reaction inert gas in the method has a temperature of 110 to 160°C and is fed at a pressure, in absolute pressure, of 0.08 to 0.2 MPa.

As shown in Figure 4, in a preferred embodiment of the method of the present application, lactic acid feedstock 100 is fed through the feedstock inlet between the rectification section 411 and the reaction section 412, and then enters the reaction section 412, to generate lactide under the action of a catalyst. The reaction inert gas 200 is introduced through the gas inlet into the bottom of the reaction section 412, and passes upward through the reaction section 412, stripping a portion of water and a portion of unreacted lactic acid in the reaction system to the rectification section 411. In the rectification section 411, lactic acid is separated. The mixture 400 of water vapor and inert gas enters the condenser 413 from the top of the apparatus for separation. After condensation, part of the liquid phase component 500, which is mainly water, is refluxed and part of the liquid phase component 500 is discharged from the system. The recovered inert gas 600 is dried, heated and returned to the bottom of the catalytic distillation apparatus. Crude lactide product 300 is withdrawn from the bottom of the reaction section 412.

With the method of the present application, the composition of the crude lactide product withdrawn from the bottom of the reaction section, by weight, can reach 76.0-86.0% L-lactide, ≤2.0% m-lactide, 1.0-4.0% L -lactic acid, 1.0-4.0% lactic acid dimer, 1.0-6.0% lactic acid trimer, and 1.0-8.0% lactic acid multimer. The single-pass yield of lactide in the synthesis process can reach 80% or higher.

In a preferred embodiment, the method of the present application further includes the step of pretreating and purifying the crude lactide product to obtain the lactide product. The pretreatment and purification of the crude lactide product can be performed in a manner well known to those skilled in the art.

In some further preferred embodiments, the pretreatment involves rinsing the crude lactide with a mixture of water and ethanol to remove unreacted lactic acid and multimers. The rinsing temperature is 0-10 °C to reduce the hydrolysis rate of lactide by water and the solubility of lactide in ethanol, thereby increasing the product yield.

In some further preferred embodiments, the pretreatment is performed by rinsing the crude lactide with a dilute alkaline solution followed by rinsing with cold water to remove unreacted lactic acid and lactic acid multimers, with a rinsing temperature of 0-10 °C.

In the two types of further preferred embodiments described above, after being rinsed, the crude lactide is then further separated by filtration and centrifugation before being sent to a cyclone dryer or a rotary drum vacuum dryer for drying.

In a preferred embodiment, after pretreatment of crude lactide, the lactide yield can reach 92.0% or higher, and the purity of L-lactide can reach 92% or higher, meeting the requirements for subsequent refining and purification.

In a preferred embodiment, the refining and purification involves further processing the pretreated lactide through melt crystallization or distillation to obtain the lactide product, thereby meeting the polymerization requirements.

### Examples

The present application will be further described in detail below with reference to the examples, but the present application is not limited to these examples.

The experiments in the following examples were carried out in the catalytic distillation apparatus shown in Figure 4, in which the catalytic distillation column had a height of 1.8 m and a diameter of 80 mm, and the lower reaction section thereof was packed with multiple layers of structured catalytic packing assembled from catalytic packing modules and packing modules. In the catalytic packing module, the first mesh layer was made of polyvinylidene fluoride wire mesh, with circular mesh openings, a porosity of 60.0%, and a mesh size of 14 mesh; the second mesh layer was a wire mesh made of glass fiber fabric, with circular mesh openings, a porosity of 65.0%, and a mesh size of 18 mesh; and the inert packing material was ceramic rings with a particle size of 1.6 mm. The packing modules were stainless steel wire mesh corrugated packing. The catalytic packing modules and the packing modules were alternately arranged in the horizontal direction as shown in Figure 2 or Figure 3 to assemble the structured catalytic packing, and multiple layers were installed in the vertical direction in the manner shown in Figure 2 or Figure 3 to fill the entire reaction section of the catalytic distillation apparatus. The upper rectification section of the catalytic distillation apparatus was packed with structured packing assembled from titanium wire mesh corrugated packing material.

Unless otherwise specified, the experimental methods used in the following examples and comparative examples are conventional methods in the art; unless otherwise specified, the experimental materials used can be purchased from biochemical reagent stores.

The lactic acid used in the following examples and comparative examples is heat-resistant L-lactic acid with an optical purity of not less than 99.0%.

In the following examples and comparative examples, an Agilent high-performance liquid chromatograph was used to analyze the chemical purity of lactide. A UV detector was used, and phosphoric acid and acetonitrile were employed as the mobile phase. The chromatographic column was a ZORBAX SB-Aq, with a length of 250 mm, an inner diameter of 4.6 mm, and a packing particle size of 5 µm. The detection wavelength was 200 nm, the column temperature was 40 °C, the flow rate was 1 mL/min, and the injection volume was 5 µL.

In the following examples and comparative examples, an Agilent gas chromatograph was used to analyze the content of different optical isomers of lactide. A CYCLOSIL-B column was selected, with a vaporization chamber temperature of 250 ° C, a detector temperature of 280°C, and a hydrogen flame ionization detector. The column temperature program was as follows: initial temperature 100°C, held for 5 min, increased to 140°C at a rate of 4°C/min, held for 7 min, increased to 200°C at a rate of 8°C/min, and held for 20 min. The carrier gas flow rate was 1.4 mL/min for N₂, 30 mL/min for hydrogen, and 400 mL/min for air. The injection volume was 0.5 µL.

In the following examples and comparative examples, the yield Y of the one-step liquid-phase cyclization process for preparing lactide from lactic acid is calculated using the following formula: $Y = \frac{m}{{m}_{0} \times {x}_{0} \times \frac{{M}_{lactide}}{2 {M}_{lactic acid}}} \times 100 %$

Where *m*₀ is the lactic acid feed rate, *x*₀ is the content of pure lactic acid in the feed, M_{lactide} is the molecular weight of lactide, M_{lactic acid} is the molecular weight of lactic acid, and *m* is the L-lactate production rate.

### Example 1

A 50wt% L-lactic acid aqueous solution was heated to 90°C and continuously fed into the catalytic distillation apparatus at a flow rate of 2.0 kg/h. The rectification section had a theoretical plate number of 6, a condensation temperature of the top condenser of 20°C, an overhead temperature of 125 °C, an overhead pressure of 0.08 MPa absolute, and a reflux ratio of 1: 3. The reaction section was packed with 12 layers of structured catalytic packing (corresponding to 6 theoretical plates), using the packing manner shown in Figure 2. The catalytic packing modules on the structured catalytic packing were catalytic packing modules filled with Sn/Ti- β molecular sieve catalyst. The catalyst was in the form of spherical particles with a particle size of 2 mm. Based on the total reaction section volume, the catalyst loading amount in the catalytic packing module was 0.40 cm³ catalyst/cm³ reaction section volume, and the inert packing material loading in the catalytic packing module was 0.15 cm³ inert packing material/cm³ reaction section volume. The reaction pressure was 0.1 MPa, and the reaction temperature was 140 °C. The residence time of the material in the reaction section was 40 min. N₂ was introduced into the bottom of the reaction section as a stripping gas at a flow rate of 5.0 L/h, with a temperature of 140°C and a pressure of 0.15 MPa. The crude lactide product formed was withdrawn from the bottom of the reaction section at a flow rate of 0.80 kg/h.

Through analytical calculation, the single-pass yield of lactide in the above catalytic distillation reaction process could reach 83.1%. In the withdrawn crude lactide product, the content of L-lactide was 84.3%, the content of m-lactide was 1.2%, the content of L-lactic acid was 2.1%, the content of lactic acid dimer was 2.7%, the content of lactic acid trimer was 3.6%, and the content of lactic acid multimers (tetramer and above) was 6.3%.

The withdrawn crude lactide was fed into a centrifuge and subjected to alkaline washing with 0.1 mol/L sodium hydroxide, then rinsed with water, and subsequently dried in a rotary drum vacuum dryer at 40°C for 8.0 h to obtain pre-treated lactide with a product purity of 93.7%. Thereafter, the lactide was further purified by melt crystallization to obtain a lactide product meeting the requirements for polymerization.

### Example 2

A 30wt% L-lactic acid aqueous solution was heated to 90°C and continuously fed into the catalytic distillation apparatus at a flow rate of 2.0 kg/h. The rectification section had a theoretical plate number of 8, a condensation temperature of the top condenser of 10°C, an overhead temperature of 105 °C, an overhead pressure of 0.05 MPa absolute, and a reflux ratio of 1: 5. The reaction section was packed with 16 layers of structured catalytic packing (corresponding to 8 theoretical plates), using the packing manner shown in Figure 3. The catalytic packing modules on the structured catalytic packing were catalytic packing modules filled with Sn/Ti- β molecular sieve catalyst. The catalyst was in the form of cylindrical particles with a diameter of 2 mm and a length of 4 mm. Based on the total reaction section volume, the catalyst loading amount in the catalytic packing module was 0.40 cm³ catalyst/cm³ reaction section volume, and the inert packing material loading in the catalytic packing module was 0.10 cm³ inert packing material/cm³ reaction section volume. The reaction pressure was 0.08 MPa, and the reaction temperature was 120 °C. The residence time of the material in the reaction section was 30 min. N₂ was introduced into the bottom of the reaction section as a stripping gas at a flow rate of 4.0 L/h, with a temperature of 120°C and a pressure of 0.15 MPa. The crude lactide product formed was withdrawn from the bottom of the reaction section at a flow rate of 0.49 kg/h.

Through analytical calculation, the single-pass yield of lactide in the above catalytic distillation reaction process could reach 83.9%. In the withdrawn crude lactide product, the content of L-lactide was 81.8%, the content of m-lactide was 1.0%, the content of L-lactic acid was 2.4%, the content of lactic acid dimer was 3.5%, the content of lactic acid trimer was 5.7%, and the content of lactic acid multimers (tetramer and above) was 5.1%.

The withdrawn crude lactide was separated by centrifugation, and the solid powder was then sequentially rinsed with water and ethanol at a rinsing temperature of 10°C. Subsequently, it was transferred to a vacuum dryer and dried at 40°C for 6.0 h to obtain pre-treated lactide with a product purity of 94.5%. Thereafter, the lactide was further purified by melt crystallization to obtain a lactide product meeting the requirements for polymerization.

### Example 3

A 60wt% L-lactic acid aqueous solution was heated to 100°C and continuously fed into the catalytic distillation apparatus at a flow rate of 2.0 kg/h. The rectification section had a theoretical plate number of 4, a condensation temperature of the top condenser of 10°C, an overhead temperature of 135 °C, an overhead pressure of 0.1 MPa absolute, and a reflux ratio of 1: 2. The reaction section was packed with 8 layers of structured catalytic packing (corresponding to 4 theoretical plates), using the packing manner shown in Figure 2. The catalytic packing modules on the structured catalytic packing were catalytic packing modules packed with Sn/Ti-β molecular sieve catalyst. The catalyst was in the form of spherical particles with a diameter of 2 mm. Based on the total reaction section volume, the catalyst loading amount in the catalytic packing module was 0.48 cm³ catalyst/cm³ reaction section volume, and the inert packing material loading in the catalytic packing module was 0.15 cm³ inert packing material/cm³ reaction section volume. The reaction pressure was 0.15 MPa, and the reaction temperature was 160 °C. The residence time of the material in the reaction section was 20 min. N₂ was introduced into the bottom of the reaction section as a stripping gas at a flow rate of 6.0 L/h, with a temperature of 160°C and a pressure of 0.15 MPa. The crude lactide product formed was withdrawn from the bottom of the reaction section at a flow rate of 0.97 kg/h.

Through analytical calculation, the single-pass yield of lactide in the above catalytic distillation reaction process could reach 81.2%. In the withdrawn crude lactide product, the content of L-lactide was 80.3%, the content of m-lactide was 1.9%, the content of L-lactic acid was 1.7%, the content of lactic acid dimer was 2.8%, the content of lactic acid trimer was 4.6%, and the content of lactic acid multimers (tetramer and above) was 7.9%.

The withdrawn crude lactide was separated by centrifugation, and the solid powder was then sequentially rinsed with water and ethanol at a rinsing temperature of 10°C. Subsequently, it was transferred to a vacuum dryer and dried at 40°C for 6.0 h to obtain pre-treated lactide with a product purity of 94.1%. Thereafter, the lactide was further purified by melt crystallization to obtain a lactide product meeting the requirements for polymerization.

### Example 4

The experiment was carried out with reference to Example 1, except that the catalytic packing modules on the structured catalytic packing were catalytic packing modules packed with Sn-β molecular sieve catalyst (i.e., a β molecular sieve catalyst loaded with Sn only).

The withdrawn amount of the crude lactide at the bottom of the reaction section of the catalytic distillation apparatus was 0.82 kg/h, and the single-pass yield of lactide could reach 81.5%. In the withdrawn crude lactide product, the content of L-lactide was 79.4%, the content of m-lactide was 1.4%, the content of L-lactic acid was 2.6%, the content of lactic acid dimer was 3.7%, the content of lactic acid trimer was 5.7%, and the content of lactic acid multimers (tetramer and above) was 7.0%.

### Example 5

The experiment was carried out with reference to Example 1, except that the catalytic packing modules on the structured catalytic packing were catalytic packing modules packed with Ti-β molecular sieve catalyst (i.e., a β molecular sieve catalyst loaded with Ti only).

The withdrawn amount of the crude lactide at the bottom of the reaction section of the catalytic distillation apparatus was 0.83 kg/h, and the single-pass yield of lactide could reach 81.0%. In the withdrawn crude lactide product, the content of L-lactide was 78.2%, the content of m-lactide was 1.6%, the content of L-lactic acid was 2.9%, the content of lactic acid dimer was 3.8%, the content of lactic acid trimer was 5.7%, and the content of lactic acid multimers (tetramer and above) was 7.5%.

### Comparative Example 1

Experiment was carried out using a glass tank reactor to produce lactide from lactic acid via a one-step liquid-phase process. The raw materials, catalyst, reaction temperature, and pressure employed in the experiment were the same as in Example 1. The reaction equipment was a three-necked glass flask, and the reaction was carried out in an oil bath. 50 mL of lactic acid feedstock and 100 mL of toluene were added together to the glass flask, and the reaction was carried out at 125°C for 6.0 h. A condensing separator was used to separate the water and toluene distilled off the reaction mixture into layers, with the toluene being refluxed back into the reaction system as a water-entraining agent.

After the reaction was completed, the bottom sample was taken and analyzed, wherein the content of L-lactide was 74.5%, the content of m-lactide was 1.7%, the content of L-lactic acid was 2.5%, the content of lactic acid dimer was 2.9%, the content of lactic acid trimer was 4.6%, and the content of lactic acid multimers (tetramer and above) was 14.6%. The lactide yield was 63.2%.

### Comparative Example 2

The raw materials, catalyst, loadings of catalyst and inert packing, and reaction process conditions, etc., were referred to those in Example 1, except that in the structured catalytic packing within the reaction section of the catalytic distillation apparatus, the catalytic packing modules were in the form of catalyst and inert packing material uniformly mixed and then wrapped with metal wire mesh. The catalytic packing modules and the packing modules were arranged alternately in the horizontal direction, while an upper layer of structured catalytic packing and a lower layer of structured catalytic packing were arranged in the staggered manner as in Example 1. The crude lactide product was also withdrawn from the bottom of the reaction section.

Through analysis, in the withdrawn crude lactide product, the content of L-lactide was 75.9%, the content of m-lactide was 1.3%, the content of L-lactic acid was 2.3%, the content of lactic acid dimer was 3.9%, the content of lactic acid trimer was 6.2%, and the content of lactic acid multimers (tetramer and above) was 10.1%. The single-pass lactide yield was only 69.4%.

### Comparative Example 3

The raw materials, catalyst, catalyst loading amount and reaction process conditions, etc., were referred to those in Example 1, except that in the reaction section of the catalytic distillation apparatus, the catalyst and inert packing material were uniformly mixed and then loaded into the entire reaction section. Sieve plate support structures were provided at the upper and lower ends of the catalytic reaction section, respectively, to secure the catalytic reaction zone and prevent entrainment of the catalyst by the gas phase. The catalyst loading amount was 0.40 cm³ catalyst/cm³ reaction section volume, and the inert packing material loading was 0.60 cm³ inert packing material/cm³ reaction section volume. Crude lactide product was also withdrawn from the bottom of the reaction section.

Through analysis, in the withdrawn crude lactide product, the content of L-lactide was 72.7%, the content of m-lactide was 1.2%, the content of L-lactic acid was 5.2%, the content of lactic acid dimer was 4.5%, the content of lactic acid trimer was 5.9%, and the content of lactic acid multimers (tetramer and above) was 10.3%. The single-pass lactide yield was only 60.3%.

### Comparative Example 4

The raw materials, catalyst, loadings of catalyst and inner packing and reaction process conditions, etc., were referred to those in Example 1, except that the reaction section of the catalytic distillation apparatus adopted a layered loading form of catalytic packing layer and structured packing layer. That is, a layer of mixed catalyst and inert packing material wrapped with metal wire mesh, and a layer of structured packing, the two were arranged alternately. A sieve plate support structure was provided at the lower end of the catalytic reaction section. Crude lactide product was also withdrawn from the bottom of the reaction section.

Through analysis, in the withdrawn crude lactide product, the content of L-lactide was 75.4%, the content of m-lactide was 1.5%, the content of L-lactic acid was 2.2%, the content of lactic acid dimer was 4.7%, the content of lactic acid trimer was 5.9%, and the content of lactic acid multimers (tetramer and above) was 10.1%. The single-pass lactide yield was only 70.9%.

Comparing the results of Examples 1-5 and Comparative Example 1, it is clear that by using the catalytic distillation apparatus and process of the present application, the product quality can be guaranteed and the product yield can be improved while achieving continuous operation of the reaction process.

Comparing the results of Examples 1-5 with Comparative Examples 2, 3 and 4, it is evident that using a catalytic distillation apparatus with a reaction section packed with the structured catalytic packing of the present application can effectively improve reaction efficiency, reduce the re-hydrolysis of lactide generated in the reaction, and significantly increase the single-pass yield of the reaction.

The preferred embodiments of the present application have been described in detail above. However, the present application is not limited to the specific details of the above embodiments. Within the scope of the technical concept of the present application, various simple modifications can be made to the technical solution of the present application, and these simple modifications all fall within the protection scope of the present application.

It should also be noted that the various specific technical features described in the above embodiments can be combined in any suitable manner without contradiction. In order to avoid unnecessary repetition, the present application will not describe the various possible combinations separately.

Furthermore, various different embodiments of the present application can be combined in any way, as long as they do not violate the spirit of the present application, they should also be regarded as the content disclosed in the present application.

## Claims

1. A catalytic packing module suitable for a catalytic distillation apparatus, comprising, sequentially from the inside out, a solid catalyst layer at the center, a first mesh layer wrapped around the outside of the catalyst layer, an inert packing layer wrapped around the outside of the first mesh layer, and a second mesh layer wrapped around the outside of the inert packing layer.

2. The catalytic packing module according to claim 1, wherein the first mesh layer and the second mesh layer are made of corrosion-resistant materials having different hydrophilic and/or lipophilic properties;
preferably, the first mesh layer is made of a hydrophobic and corrosion-resistant material, such as a polyester, a fluoropolymer or a metal composite material, particularly is made of a polyvinylidene fluoride; and the second mesh layer is made of a highly permeable and hydrophilic corrosion-resistant material, such as a highly permeable hydrophilic fiber coalescence material, particularly glass fiber fabric.

3. The catalytic packing module according to claim 1 or 2, having one or more of the following features:
the first mesh layer and the second mesh layer are each independently in the form of a corrugated mesh or a wire mesh;
the porosity of the first and second mesh layers is independently 50.0-70.0%, and the mesh size is independently 7-30 mesh, preferably 10-25 mesh;
the inert packing is selected from glass beads, ceramic rings, ceramic spheres, ceramic skeletons, quartz sand, silica sand, bentonite, or combinations thereof, preferably with a particle size of the packing of 1.0-2.0 mm; and
the catalyst is in the form of spheres or porous spheres having a diameter of 1-5 mm, cylinders or porous cylinders having a diameter of 0.5-3 mm and a length of 1-5 mm, or cloverleaf shapes or polyhedral shapes having an equivalent diameter of 1-5 mm.

4. The catalytic packing module according to any one of claims 1-3, wherein the catalyst is a catalyst based on a molecular sieve loaded with Sn and/or Ti, preferably a catalyst based on a β molecular sieve loaded with Sn and/or Ti, particularly preferably a catalyst of a β molecular sieve loaded with Sn and Ti.

5. A structured catalytic packing, comprising catalytic packing modules and packing modules arranged alternately in a horizontal direction, wherein the catalytic packing modules are the catalytic packing modules according to any one of claims 1-4,
preferably, the packing modules are composed of stainless steel wire mesh corrugated packing.

6. A catalytic distillation apparatus, comprising an upper rectification section and a lower reaction section, with a feed inlet provided between the upper rectification section and the lower reaction section, wherein the lower reaction section is packed with at least one layer of the structured catalytic packing according to claim 5,
preferably, the lower reaction section of the catalytic distillation apparatus is packed with multiple layers, preferably 4-16 layers, of the structured catalytic packing according to claim 5, and in the vertical direction, the catalytic packing modules and the packing modules of two adjacent layers of the structured catalytic packing among the multiple layers are arranged in a staggered manner vertically.

7. The catalytic distillation apparatus according to claim 6, wherein the upper rectification section of the catalytic distillation apparatus is packed with a structured packing selected from titanium wire mesh corrugated packing, perforated plate corrugated packing, or a combination thereof.

8. The catalytic distillation apparatus according to claim 6 or 7, further comprising a condenser disposed at the top, and a gas inlet and a product outlet disposed at the bottom of the reaction section;
optionally, a feed distributor is provided at the feed inlet position of the catalytic distillation apparatus, and/or a gas distributor is provided at the gas inlet position; and
optionally, a reboiler is further provided at the bottom of the catalytic distillation apparatus.

9. Use of the catalytic packing module according to any one of claims 1 to 4, the structured catalytic packing according to claim 5, or the catalytic distillation apparatus according to any one of claims 6 to 8 in a solid-phase catalytic reaction of a liquid feedstock, particularly in a reaction process for preparing lactide from lactic acid via a one-step liquid-phase method,
preferably, the first mesh layer and the second mesh layer of the catalytic packing module are made of corrosion-resistant materials having different hydrophilic and/or lipophilic properties;
further preferably, the first mesh layer of the catalytic packing module is made of a hydrophobic and corrosion-resistant material, such as a polyester, a fluoropolymer, or a metal composite material, particularly is made of a polyvinylidene fluoride; and the second mesh layer of the catalytic packing module is made of a highly permeable and hydrophilic corrosion-resistant material, such as a highly permeable hydrophilic fiber coalescence material, particularly glass fiber fabric.

10. A catalytic distillation reaction method, comprising the steps of: in the reaction section of the catalytic distillation apparatus according to any one of claims 6 to 8, bringing a liquid feedstock into contact with a solid catalyst arranged within the catalytic packing module for reaction, and separating a resulting reaction product in the rectification section;
preferably, the first mesh layer and the second mesh layer of the catalytic packing module are made of corrosion-resistant materials having different hydrophilic and/or lipophilic properties.

11. A one-step continuous process for preparing lactide, comprising the step of: in the catalytic distillation apparatus according to claim 8, bringing lactic acid into contact with a catalyst for reaction to obtain lactide, wherein a feedstock containing lactic acid enters through the feed inlet of the catalytic distillation apparatus, comes into contact with the catalyst within the catalytic packing modules in the reaction section for reaction, an reaction inert gas enters through the gas inlet at the bottom of the reaction section and passes upward through the reaction section, stripping a portion of water and a portion of unreacted lactic acid from the reaction materials in the reaction section into the rectification section, and a crude lactide product is withdrawn from the product outlet at the bottom of the reaction section,
preferably, the first mesh layer of the catalytic packing module is made of a hydrophobic and corrosion-resistant material, such as a polyester, a fluoropolymer, or a metal composite material, particularly is made of a polyvinylidene fluoride; and the second mesh layer of the catalytic packing module is made of a highly permeable and hydrophilic corrosion-resistant material, such as a highly permeable hydrophilic fiber coalescence material, particularly glass fiber fabric.

12. The process according to claim 11, having one or more of the following features:
the rectification section of the catalytic distillation apparatus has a theoretical plate number of 4 to 8;
the reflux ratio of the rectification section of the catalytic distillation apparatus is from 2:1 to 1:5; and
the reaction section of the catalytic distillation apparatus has a theoretical plate number of 2 to 8.

13. The process according to claim 11 or 12, having one or more of the following features:
the catalyst loading amount in the catalytic packing modules in the reaction section of the catalytic distillation apparatus is 0.1 to 0.6 cm³, preferably 0.2 to 0.6 cm³ of catalyst volume per cm³ of reaction section volume;
the inert packing material loading amount in the catalytic packing modules in the reaction section of the catalytic distillation apparatus is 0.05 to 0.3 cm³, preferably 0.1 to 0.3 cm³ of inert packing material per cm³ of reaction section volume;
the pressure in the reaction section of the catalytic distillation apparatus is, in absolute pressure, from 0.05 to 0.15 MPa, preferably 0.08 to 0.12 MPa;
the reaction temperature in the reaction section of the catalytic distillation apparatus is from 100 to 160°C, preferably 120 to 140°C, and the residence time of the feedstock containing lactic acid in the reaction section is from 20 to 60 min; and
the operating conditions in the rectification section of the catalytic distillation apparatus comprise: an overhead temperature of 95 to 140°C, and an overhead pressure, in absolute pressure, of 0.02 to 0.1 MPa.

14. The process according to any one of claims 11 to 13, having one or more of the following features:
the feedstock containing lactic acid is a 30 to 60 wt% aqueous lactic acid solution, with a feed temperature of 80 to 100°C; and
the reaction inert gas has a temperature of 110 to 160°C and is fed at a pressure, in absolute pressure, of 0.08 to 0.2 MPa, which is greater than the reaction pressure in the reaction section.
